# EUROPEAN PATENT APPLICATION

(11) **EP 1 566 183 A1**
(43) Date of publication of application: **24.08.2005**
(21) Application number: 04003950.5
(22) Date of filing: 20.02.2004
(51) Int. Cl.: A61K 38/45, A61P 9/00

(54) **Use of factor XIII for stimulating the perfusion of ischemic tissue**

(71) Applicant: ZLB Behring GmbH, 35041 Marburg (DE)
(72) Inventor: Prof.Dr. Inbal, Aida, 45 267 Hod Hasharon (IL); Dr. Dardik, Rima, Rishon Le Zion 75544 Israel (IL); Prof. Leor, Jonathan, 55900 Gane-Tikva (IL); Prof.Dr.Dickneite, Gerhard, 35043 Marburg (DE)

(57) **Abstract**

Subject of the present invention is the use of a Factor XIII-preparation preferably in injectable form for the treatment of diseases which are associated with disturbed blood perfusion of the tissue following transient or permanent ischemia.

## Description

Subject of the present invention is the use of a Factor XIII-preparation preferably in injectable form for the treatment of diseases which are associated with disturbed blood perfusion of the tissue following transient or permanent ischemia.

Angiogenesis is the process of formation of new capillaries from pre-existing blood vessels and is an essential process in embryonic development, normal physiological growth, wound healing, and tumor expansion. The process of angiogenesis consists of several steps, which include the stimulation of endothelial cells by growth factors, degradation of the extracellular matrix by proteolytic enzymes, migration and proliferation of endothelial cells, and, ultimately, capillary tube formations. Inhibition of endothelial cell apoptosis to promote cell survival is also considered to be essential for angiogenesis. Endothelial cell migration and proliferation are critical steps in the angiogenic process.

Factor XIII (FXIII) is a plasma transglutaminase that stabilizes fibrin clots in the final stages of blood coagulation. Thrombin-activated FXIII catalyzes formation of covalent crosslinks between γ-glutamyl and ε-lysyl residues of adjacent fibrin monomers to yield the mature clot. FXIII circulates in plasma as a heterotetramer composed of 2 A-subunits and 2 B-subunits. The A-subunit contains the active site of the enzyme and is synthesized by hepatocytes, monocytes, and megakaryocytes. The B-subunit serves as a carrier for the catalytic A-subunit in plasma and is synthesized by the liver.

The FXIII A-subunit gene belongs to the transglutaminase family, which comprises at least 8 tissue transglutaminases. These enzymes crosslink various proteins and are involved in many physiological and pathological process, such as hemostasis, wound healing, tumor growth, skin formation, and apoptosis. Similar to tissue transglutaminases, FXIII participates in tissue remodelling and wound healing, as can be inferred from a defect in wound repair observed in patients with inherited FXIII deficiency. FXIII also participates in implantation of the embryo during normal pregnancy; women homozygous for FXIII deficiency experience recurrent miscarriages.

Wound healing as well as embryo implantation are complex processes that involve cell proliferation and angiogenesis.

It is also known that activated FXIII (FXIIIa) supports angiogenesis by enhancing endothelial cell migration, proliferation, and survival. In an in vitro model of angiogenesis, FXIIIa significantly enhances tube formation in Matrigel. In addition, in an in vivo model FXIIIa induces new vessel formation in a rabbit cornea. The proangiogenic effect of FXIIIa is associated with downregulation of thrombospondin (TSP-1) (Dardik R., Solomon A., Loscalzo J., Eskaraev R., Bialik A., Goldberg I., Schiby G., Inbal A. Novel proangiogenic effect of Factor FXIII (FXIII) associated with suppression of thrombospondin 1 (TSP-1) expression. Arteriose Thromb Vasc Biol 2003; 23:1472-1477).

Whether Factor XIII participates in angiogenesis in ischemic tissue is not definitely known. Human recombinant tissue transglutaminase was shown to enhance angiogenesis in a rat dorsal skin flap chamber.

Thus, the problem to be solved was to analyze the effect of Factor XIII on the proliferation of new blood vessels in ischemic tissue and to examine whether or not such effect could be used for a new therapeutic preparation.

It has now been found that an injectible Factor XIII-preparation can be used for the stimulation of the perfusion of ischemic tissues by the proliferation of new blood vessels wherein the Factor XIII is activated in vitro by the addition of thrombin to generate FXIIIa. FXIII can also be activated after in vivo administration via the physiological thrombin pathway which represents the activation by the coagulation system. Alternatively topical application of a FXIII preparation to a wounded or ischemic area is possible.

Significant therapeutic effects could be expected by the use of said Factor XIII-concentrate for the treatment of myocardial infarctions, peripheral vascular diseases, ischemic strokes and non-healing ischemic wounds.
Thus, FXIII preparations could be used in all diseases which are associated with disturbed blood perfusion of the tissue following transient or permanent ischemia. It includes ischemias due to arterial or venous occlusion or obstruction of the microcirculation.
The therapeutic results of said Factor XIII-concentrate could be shown by the following methods and experiments:

### FXIII Activation

The source of FXIII was FXIII concentrate, Fibrogammin-P® (Aventis Behring). To obtain activated FXIII (FXIIIa) 2 ml of reconstituted 100 U/ml (approximately 1000 µg/ml) FXIII was incubated with thrombin immobilized on Affi-gel 10 beads. One milliliter of packed bead volume contained 200 U of thrombin. Ten millimolar CaCl₂ was added, and the mixture was incubated at 37°C for 2 hours. FXIII activation was monitored by measuring FXIII activity using a chromogenic assay (Berichrome, Dade Behring). Leakage of thrombin from the beads into the FXIII solution was excluded by the lack of color development upon addition of a thrombin-specific chromogenic substrate (S2238, Chromogenix, Sweden). FXIIIa was inactivated by treatment with 3mmol/l iodoacetamide (Sigma) for 30 minutes at 22°C to block transglutaminase activity; free iodoacetamide was then removed by dialysis.

### 1. In vivo model of rabbit cornea vascularization

New Zealand albino male rabbits (3.0 Kg in weight) were used in this study. General anesthesia was achieved by intramuscular injection of xylazine 2% and ketamine HCI. 2 µl containing either 20 µg FXIIIa or PBS were injected subepithelially in the cornea of the rabbits using a Hamilton syringe. The site of the injection was 2 mm away from the limbus, at the site of the attachment of the superior rectus muscle. In each one of the four rabbits studied, FXIIIa was injected into the right cornea and a similar volume of PBS (negative control) into the left cornea. Clinical examination of the cornea was performed prior to the injection, immediately following the injection, and then every 24 hr up to 96 hr after the maximal effect was observed. At this stage, the rabbits were sacrificed by lethal injection of pentobarbital. The corneas were excised, fixed in 4% paraformaldehyde, and stained with hematoxylin-eosin for histological evaluation by light microscopy and with GSLI - isolectin B₄ for evaluation of blood vessels.

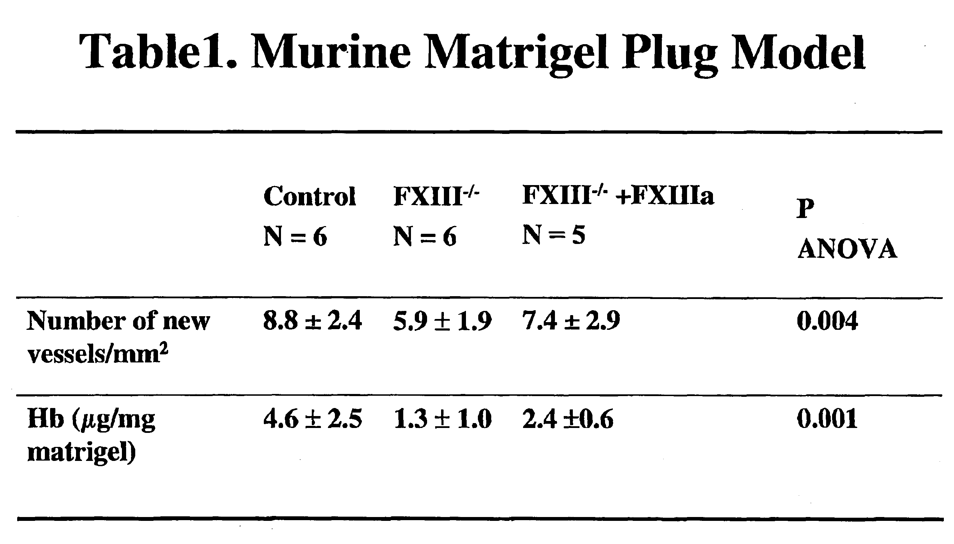

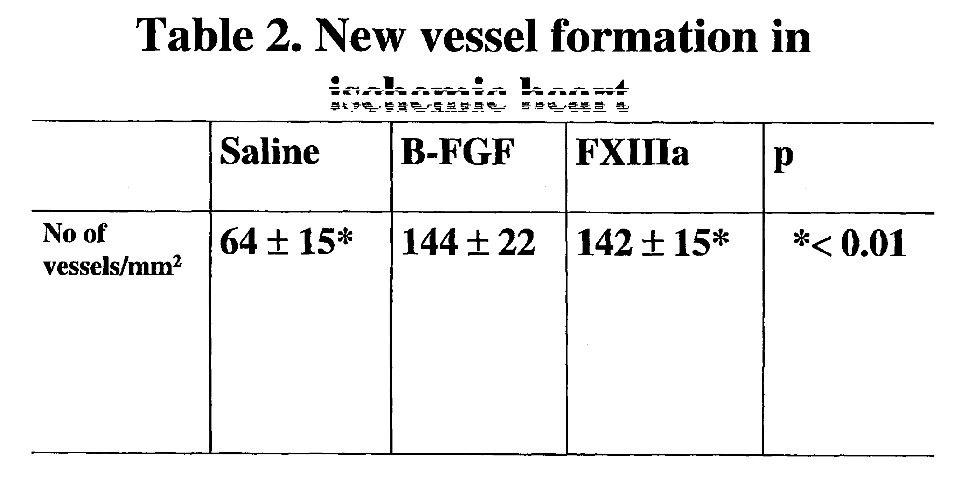

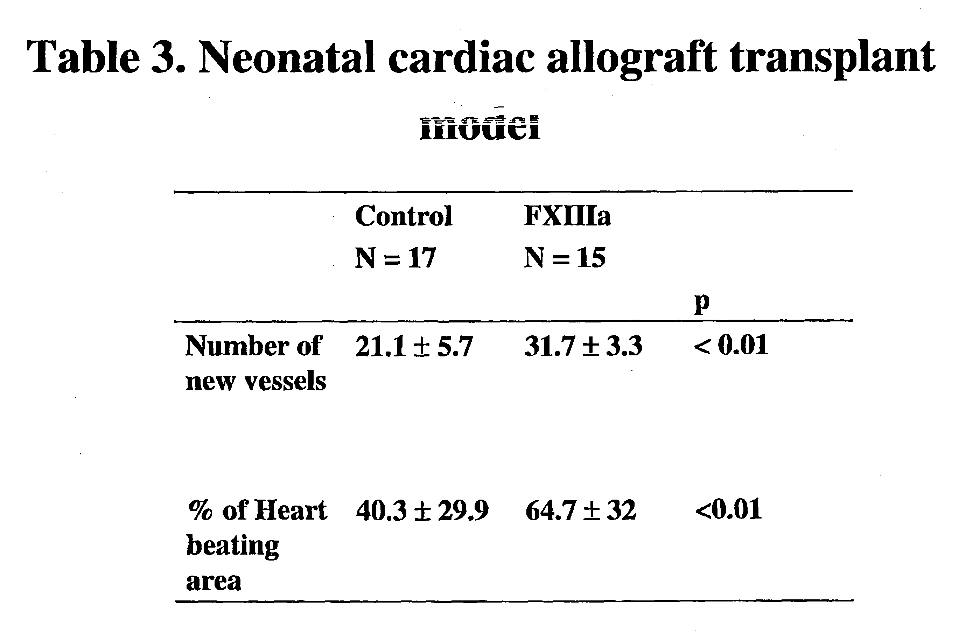

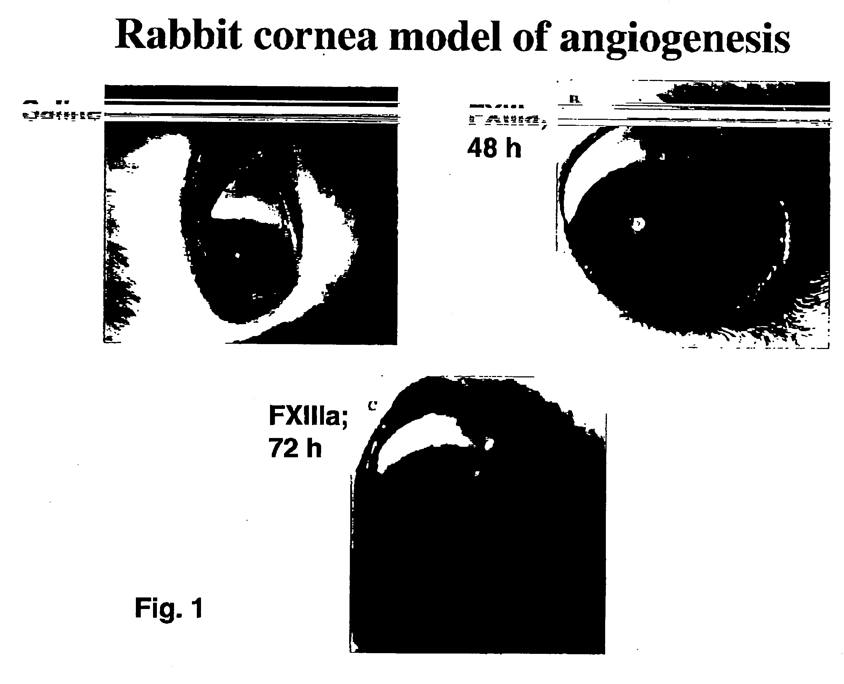

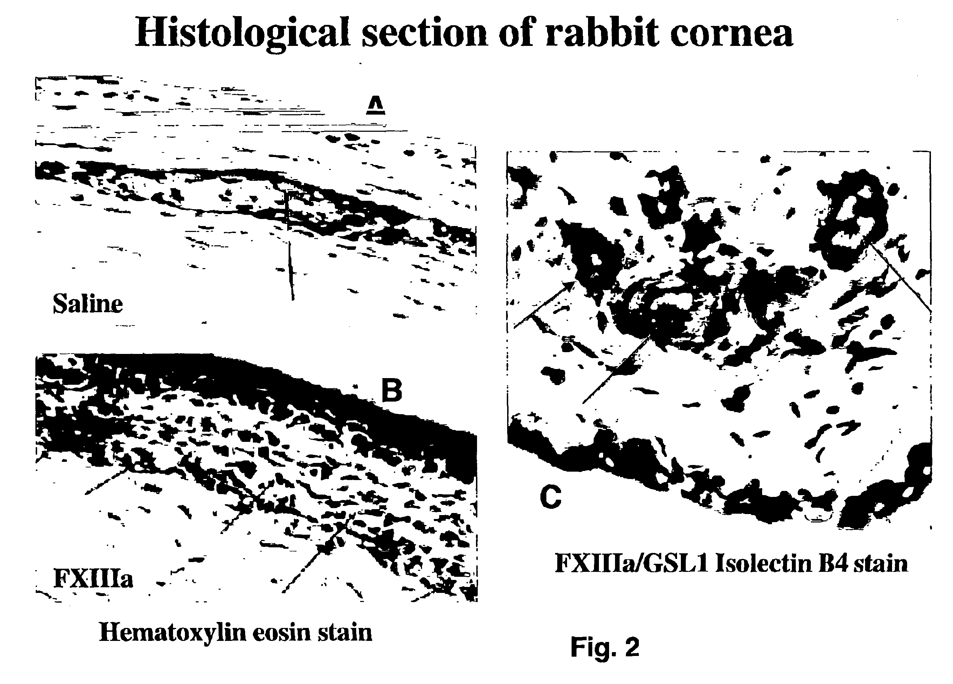

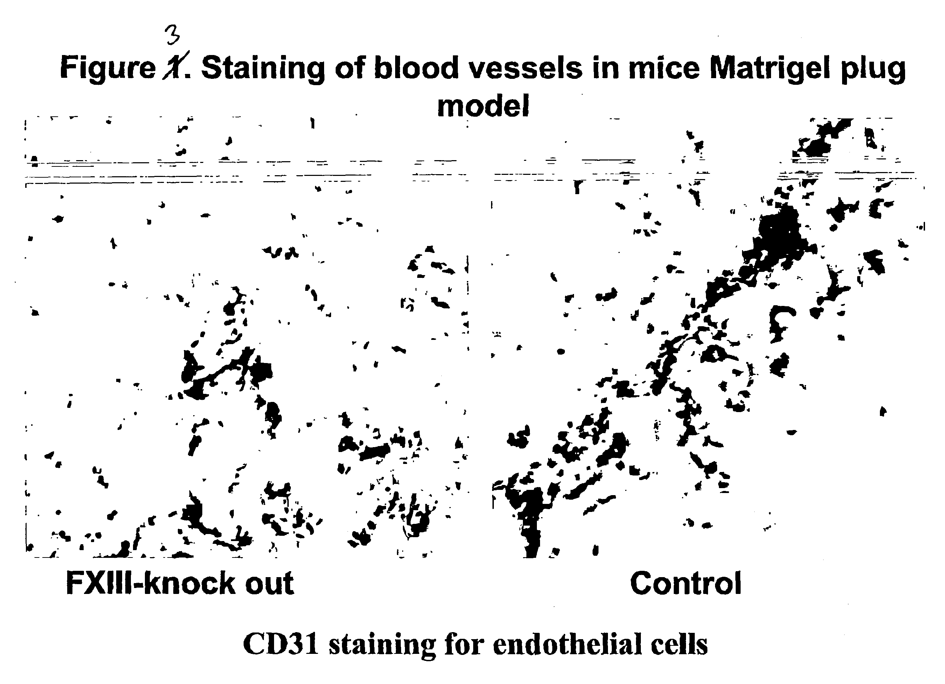

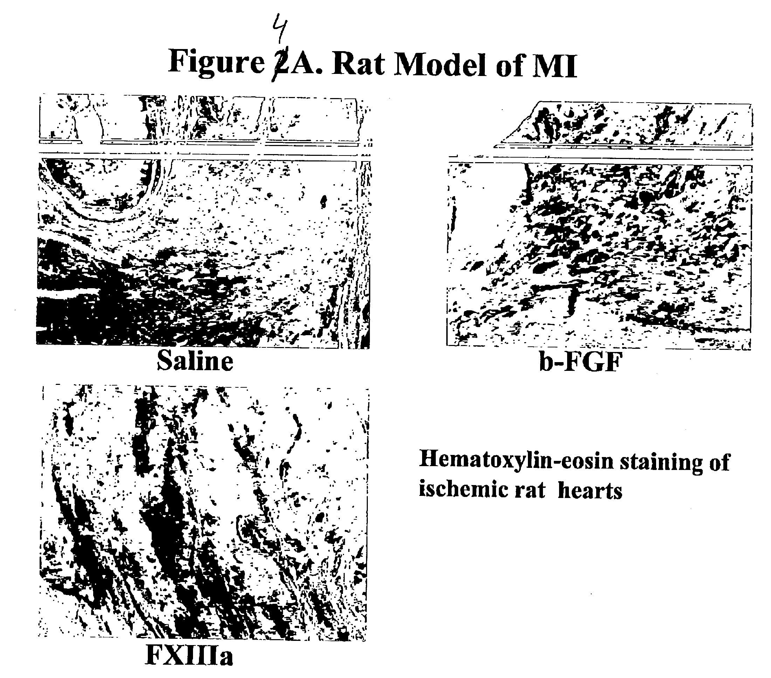

The normal cornea does not have any blood vessels (Fig. 1A). In contrast to the left eyes treated with PBS, blood vessel formation was obvious in the right eyes of all four rabbits 48 hr after injection of FXIIIa (Fig. 1 B). At 72 hr a rich network of blood vessels could be seen in the right eyes, penetrating the cornea up to 4 mm toward the center (Fig. 1 C). The development of the vascular network continued until 96 hr after FXIIIa injection. Similar results were observed in rabbits treated with bFGF. Histological section of the cornea shown in Figure 2B demonstrates the rich network of capillaries grown in the cornea of eyes treated with FXIII, as opposed to those treated with PBS (Fig. 2A). Staining of the FXIIIa -treated sections of the cornea with endothelial cell marker isolectin B₄ showed positive stain in the blood vessels (2C).

### 2. Angiogenesis in Factor XIII-knock out mice

Control (n = 6), FXIII-knock outs (n = 6) and FXIII-knock out mice treated with FXIII (n = 6) were anesthetized with an intraperitoneal injection of pentobarbital (50 mg/kg). A sterile mix of Matrigel (Becton Dickinson) (0.5 ml), heparin (20 units/ml), and bFGF (200 ng/ml), with or without FXIIIa (Fibrogammin®; 10-20 units) was injected subcutaneously. At the end of two weeks mice were euthanized. The Matrigel plug was dissected from the subcutaneous tissue and analyzed after staining with hematoxylin-eosin for histological evaluation by light microscopy and with GSLI - isolectin B4 for evaluation of blood vessels. In addition, haemoglobin of the vessels grown into the matrigel plaque was measured.

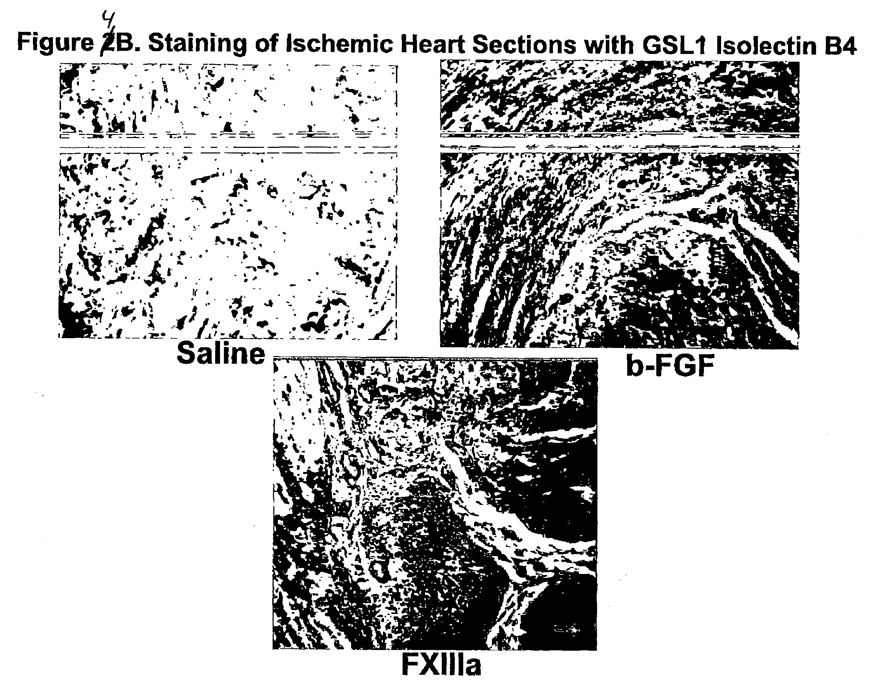

The FXIII activity in plasma of mice FXIII -/- measured by Berichrom assay was undetectable. Histological analyses of the matrigel sections showed significantly increased numbers of new vessels in the control mice compared to that of the knock out mice. In a representative picture shown in Fig.3 the amount of new vessels formed in control animals was significantly increased. The number of new vessels in the entire group of FXIII -/- mice was significantly decreased compared to that of control mice: 5.9 ± 1.9 vs. 8.8 ± 2.4, and the number increased after FXIII treatment to 7.4 ± 2.9, p = 0.004 (Table 1). The values of haemoglobin measured from the vessels/matrigel tissue (reflecting the number of blood vessels containing red blood cells) were significantly increased in the control group 4.6 ± 2.5 µg/mg matrigel vs. 1.3 ± 1.0 µg/mg matrigel in the FXIII knock out mice and the haemoglobin increased by almost 2-fold in the FXIII knock out mice treated with FXIII concentrate, p = 0.001 (Table 1)

### 3. Pro-angiogenic effect of Factor FXIII on ischemic tissue (myocardial infarction)

After ligation of coronary artery of the rat saline, bFGF or 5 units of FXIIIa were injected locally every 7 days by canula into the tissue supplied by the ligated vessel as described previously. Three weeks later the animals were sacrificed and the cardiac tissue underwent histological analysis and quantitation of the number of blood vessels. The tissue was fixed in 4% paraformaldehyde, stained with hematoxylin-eosin for histological evaluation by light microscopy and with GSLI - isolectin B4 for evaluation of blood vessels.

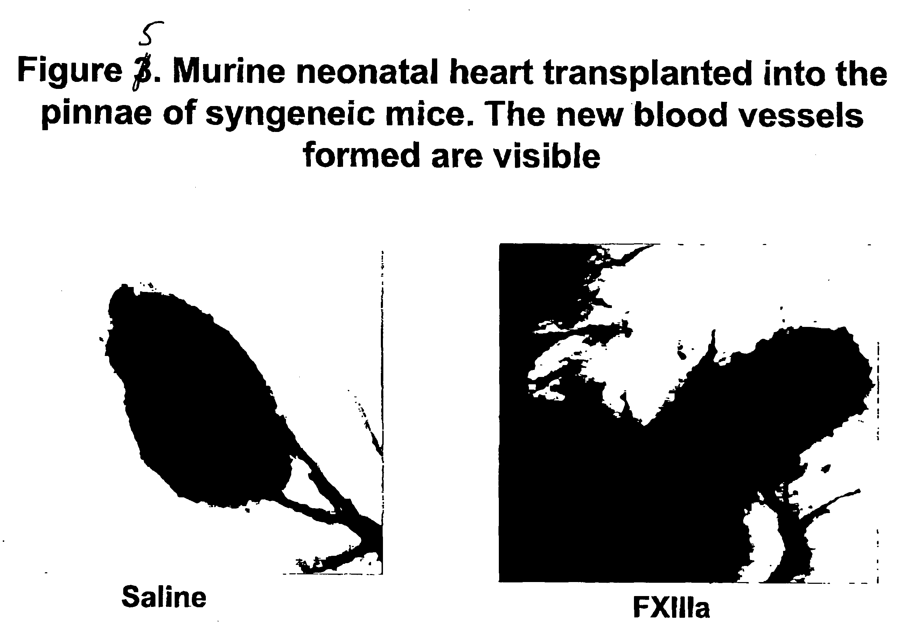

Experiments performed with 9 rats (3 treated with FXIIIa, 3 treated with bFGF and 3 treated with saline) showed that following myocardial ischemia a significant increase in new vessel formation was observed in the FXIIIa-injected as compared to saline-injected rats: 142 ± 15/mm² vs. 64 ± 15/mm²; respectively, p = < 0.001 (Table 2). The increase in new vessel formation in the bFGF-treated animals (positive control) was similar to that observed in FXIIIa-treated mice (144 ± 22/mm²; p = 0.6, Table 2). A representative picture of neovascularization of FXIIIa-, bFGF- or saline treated animals is shown in Fig. 4. None of the animals developed side effects.

### 4. The effect of Factor XIII on angiogenesis of neonatal heart transplanted into syngeneic mice

Neonatal C57BL/6N (24-hour-old) murine hearts were transplanted into the pinnae ear of syngeneic host mice. FXIIIa-treated (n = 15) or control (saline-treated, n = 17) groups were assessed one week after the engraftment by: 1 - ECG; 2 - measuring & of heart beating area; 3 - numbering of new blood vessels formation.

On day 10 the mice were sacrificed and histological analysis of the transplant cardiac tissue was undertaken with hematoxylin-eosin for histological evaluation by light microscopy and with GSLI - isolectin B4 for evaluation of blood vessels.

Thirty two senescent mice underwent transplantation with neonatal hearts: 15 were treated with FXIIIa and 17 received saline injections. As shown in Table 3, the % of the transplanted heart beating area was significantly increased in animals treated with FXIIIa: 64.7 ± 32 vs. 40.3 ± 29.9, respectively, p < 0.001. Similarly, the number of new vessels was significantly higher in the FXIIIa-treated group: 31.7 ± 3.3 vs. 21.1 ± 5.7, p < 0.01.
In addition, the number of mice with > 80% beating area was significantly higher in the FXIIIa-treated group than that in the control group: 46% vs. 17%, respectively; p = 0.038. The representative pictures of the new vessels formed in saline of FXIIIa-injected hearts are shown in Fig. 5. No side effects were observed following FXIIIa injections.

The following conclusions can be drawn from these experiments:
a) The proangiogenic effect of FXIIIa in normal and ischemic tissue is substantial
b) The new vessels formation by FXIIIa in ischemic tissue opens new therapeutic options for this compound with regard to clinical conditions where perfusion via development of new vessels is crucial, in general terms, for all diseases which are associated with disturbed blood perfusion of the tissue following transient or permanent ischemia due to venous and arterial occlusions and obstruction of the microcirculation. This includes acute myocardial infarction, peripheral vascular disease, ischemic stroke, transient ischemic attack and for treatment of non-healing ischemic wounds.
c) FXIII is commercially available as the concentrate Fibrogammin® (Aventis Behring). Following injection, FXIII can be activated by endogenous thrombin or can be injected locally in its active form after in vitro activation. Thus, feasibility and absence of side effects make FXIII an attractive potential therapeutic agent.

## Claims

1. Use of Factor XIII for the manufacture of a preparation, which is suitable for the stimulation of the perfusion of ischemic tissues.

2. Use of FXIII for the manufacture of a preparation, which is suitable for the stimulation of the perfusion of ischemic tissues by the proliferation of new blood vessels.

3. Use of FXIII as claimed in claim 1 or claim 2, wherein the FXIII is activated in vitro or in vivo.

4. Use of Factor XIII for the manufacture of an injectible preparation as claimed in claim 1, 2 or 3.

5. Use of Factor XIII for the manufacture of a preparation as claimed in claim 1, 2, 3 or 4 which is suitable for the treatment of a myocardial infarction.

6. Use of Factor XIII for the manufacture of a preparation as claimed in claim1, 2, 3 or 4, which is suitable for the treatment of an ischemic stroke or transient ischemic attack.

7. Use of FXIII for the manufacture of a preparation as claimed in claim 1, 2, 3 or 4, which is suitable for treatment of vascular or veno-occlusive diseases or obstructions of the microcirculation.

8. Use of FXIII for the manufacture of a preparation according to claims 1, 2 or 3 which is for topical application.
